Europäisches Patentamt

European Patent Office (11) Publication number: **0 025 766**

Office européen des brevets **B1**

(19)

(12) **EUROPEAN PATENT SPECIFICATION**

(45) Date of publication of patent specification: **11.07.84**

(21) Application number: **80401315.9**

(22) Date of filing: **15.09.80**

(51) Int. Cl.³: **A 61 K 31/70, A 61 K 31/785**

(54) **Pharmaceutical composition comprising modified polyriboinosinic-polyribocytidylic acid, for induction of interferon in primates.**

<table>
<tr><td>

(30) Priority: **17.09.79 US 76004**
**04.06.80 US 156295**

(43) Date of publication of application:
**25.03.81 Bulletin 81/12**

(45) Publication of the grant of the patent:
**11.07.84 Bulletin 84/28**

(84) Designated Contracting States:
**AT BE CH DE FR GB IT LI LU NL SE**

(56) References cited:
**US - A - 3 679 654**
**US - A - 4 024 222**

**CHEMICAL ABSTRACTS, vol. 64, no. 8, 11th April 1966, column 11466b, Columbus, Ohio, U.S.A., M. TSUBOI et al.: "Interaction of poly-L-lysine and nucleic acids"**
**CHEMICAL ABSTRACTS, vol. 92, no. 13, 31st March 1980, page 57, no. 104368s, Columbus, Ohio, U.S.A., I.A. BEKTEMIROV et al.: "Study of the properties of modified poly-I poly-C of different molecular weights"**

</td><td>

(73) Proprietor: **MERCK & CO. INC.**
**126, East Lincoln Avenue P.O. Box 2000**
**Rahway New Jersey 07065 (US)**

(72) Inventor: **Field, Kirk A.**
**376 Meadowbrook Road**
**North Wales, M.R.1. Pennsylvania 19454 (US)**
Inventor: **Tytell, Alfred A.**
**113 Church Road**
**Lansdale Pennsylvania 19446 (US)**
Inventor: **Lampson, George P.**
**2012 Keystone Road**
**Hatfield Pennsylvania 19440 (US)**

(74) Representative: **Corre, Jacques Denis Paul et al, Cabinet Regimbeau 26, Avenue Kléber F-75116 Paris (FR)**

(56) References cited:
**CHEMICAL ABSTRACTS, vol. 73, no. 3, 20th July 1970, page 133, no. 12103f, Columbus, Ohio, U.S.A., J.M. RICE et al.: "Enhancement by poly-D-lysine of polyinosinic acid induced interferon production in mice"**

</td></tr>
</table>

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

## Description

Background of the invention

The synthetic double-stranded RNA, polyriboinosinic-polyribocytidylic acid (poly I:C), is effective as an interferon inducer in rodents and rabbits and as such provides protection against a variety of RNA and DNA viruses in these species [A.K. Field et al., *Proc. Natl. Acad. Sci.* U.S.A., *58*, pp. 1004—1010, (1967)]. However, poly I:C shows only a very weak capacity to induce interferon in man and non-human primates.

It has been stated that enzymatic inactivation of poly I:C is the reason for its poor capacity to induce interferon in primates [H.B. Levy et al., *J. Inf. Dis., 132,* p. 434 (1975)]. These authors have reported the preparation of a solubilized formulation of poly I:C and poly-L-lysine (molecular weight 2000 to 5000) in carboxymethylcellulose (CMC) as the solubilizing agent (see also Levy, U.S. Patent 3 952 097 (1976)). This complex is relatively resistant to ribonuclease and induces significant quantities of interferon in rhesus monkeys, chimpanzees and man.

However, certain disadvantages are associated with CMC. It is a polysaccharide which is at best poorly beodegradable and on repeated injection may cause deposition of residues which may cause irritations (pathology). In addition, CMC has been considered to be a potential carcinogen. Both the sodium and ammonium salts of CMC appear on the NIOSH Suspected Carcinogen List, U.S. Environmental Protection Agency, Office of Toxic Substances, March, 1976. The references upon which these listings were made are: A. L. Walpole, *Morphol. Precursors Cancer,* Proc. Inter. Conf. Perugia, Italy, pp. 83—88, 1961 (Publ. 1962) and *Rev. Canad. Biol.* (Mont.), *20,* pp. 701 (1961).

In the Journal of Molecular Biology, volume 15 (1), pages 256—25, 1966, there are disclosed solutions of a complex of poly I:C/poly L-lysine hydrobromide at a molar ratio of poly I:C to poly L-lysine hydrobromide of 2:1. Such solutions are extremely dilute with the result that the complex does not present a solubility problem and does not induce interferon.

US—A 3 679 654 discloses complexes of nucleic acid polymers, such as poly I:C, with polycationic substances as solubilizing agents.

Summary of the invention

In the present invention a combination of poly I:C (having a high molecular weight) with poly-L-lysine hydrobromide (having a high molecular weight) have been prepared in such a molar ratio as to preclude the need for a solubilizing agent in a pharmaceutical solution of relatively high concentration. Our preparations of poly I:C/poly-L-lysine hydrobromide are superior inducers of antiviral levels of interferon in primates. The combination is prepared so that the molar ratio of poly I:C to poly-L-lysine hydrobromide (or poly-L-lysine hydrochloride) is 2 to 1 (calculated as ratio of nucleotide residue to lysine residue). Since the peptide hydrobromide is generally hygroscopic, on a moisture-free basis (of the peptide) the best complex may be prepared by mixing by weight one part of poly I:C with 0.31 parts of poly-L-lysine hydrobromide. Viscosity and solubility considerations limit the concentration of ingredients per ml that may be prepared. In practice the concentration of poly I:C per ml is used at from 1—2 mg/ml. This requires a theoretical amount of from 0.31 to 0.62 mg of dry poly-L-lysine hydrobromide, respectively. [In practice, the ratio of poly I:C to poly-L-lysine hydrobromide is such as to yield a complex at the limit of solubility]. The vehicle used is isotonic, pyrogen-free phosphate buffered saline (pH 7.2) although any suitable physiologically acceptable vehicle may be used.

The combinations of poly I:C and poly-L-lysine hydrobromide is prepared by mixing a solution of poly I:C of known concentration and suitable molecular weight with a solution of poly-L-lysine hydrobromide of known concentration and suitable molecular weight. The solutions are each prepared in phosphate buffered saline. When the two solutions are mixed a precipitate appears which will go into solution (become soluble) with mixing at room temperature or at 2—8°C in approximately 72 hours, although a small amount of undissolved solids (less than 2% of the ingredients) may be present. The solution is clarified by filtration through suitable glass or membrane filters to yield a homogeneous solution.

The solution of poly I:C is prepared by mixing solutions of equimolar quantities of the individual homopolynucleotides, poly I and poly C, to a final concentration of about 2 mg/ml. The complex is characterized prior to addition of the poly-L-lysine · HBr.

The poly I used has the following properties:

| | |
|---|---|
| Ultraviolet spectrum | Satisfactory |
| Absorption maximum | 248 nm |
| Absorption minimum | 225 nm |
| Extinction coefficient ($E_{1\%}$) at 248 nm | 240 |
| Nucleotide content | 2.4 $\mu$M/mg |
| Sedimentation coefficient (Sw, 20) | 19.2 |

The poly C used has the following properties:

| | |
|---|---|
| Ultraviolet spectrum | Satisfactory |
| Absorption maximum | 268 nm |
| Absorption minimum | 249 nm |
| Extinction coefficient ($E_{1\%}$) at 248 nm | 177 |
| Nucleotide content | 2.95 $\mu$M/mg |
| Sedimentation coefficient (Sw, 20) | 5.0 |

The poly-L-lysine · HBr used has the following properties:

| | |
|---|---|
| Molecular weight | 32,500 |
| Degree of polymerization (D.P.) | 155 residues |
| Lysine content (% of theoretical) | 89.7 (of net weight) |
| Bromide content (% of theoretical) | 87.2 (of net weight) |
| Other amino acids | None |

The poly I:C, prepared from the above poly I and poly C, has the following properties:

| | |
|---|---|
| Ultraviolet spectrum | Satisfactory |
| Absorption maximum | 248, 265 nm |
| Absorption minimum | 228 nm |
| Extinction coefficient ($E_{1\%}$) at 265 nm | 145 |
| Hypochromicity at 248 nm | 35.5% |
| Thermal denaturation midpoint (Tm) | 64°C |
| Hyperchromicity on thermal denaturation | 77.8% |
| Sedimentation coefficient (Sw, 20) | 11.0 |
| Relative viscosity | 1.59 |

The poly I:C/poly-L-lysine prepared from the above poly I, poly C, and poly-L-lysine, has the following properties:

| | |
|---|---|
| Ultraviolet spectrum | Satisfactory |
| Absorption maximum | 248, 265 nm |
| Absorption minimum | 230 nm |
| Extinction coefficient ($E_{1\%}$) at 265 nm | 140 |
| Thermal denaturation midpoint (Tm) | 83°C |
| Hyperchromicity on thermal denaturation | 109% at 83°C, <10 at 64°C |
| Sedimentation coefficient (Sw, 20) | 11.5 |
| Relative viscosity | 1.65 |
| Ribonuclease resistance compared to that of poly I:C | 14.0% |
| Conductivity | 7.1 millimhos |
| Nominal poly I:C concentration | 1.0 mg/ml |
| Actual measured poly I:C concentration | 1.0 mg/ml |
| Nominal poly-L-lysine concentration | 0.310 mg/ml |
| Actual measured poly-L-lysine concentration | 0.330 mg/ml |

There is same degree of variability in the chemical characteristics of the components, see the following table of ranges:

| | ($E_{1\%}$ at 248 nm) | (Sw, 20) | Molecular weight |
|---|---|---|---|
| Poly I | 240—280 | 11—20 | — |
| Poly C | 175—185 | 5—7 | — |
| Poly L-lysine | — | — | 10,000—70,000 preferably 10,000 to 35,000 |

The compositions prepared by this invention are useful in inducing antiviral levels of interferon in mammalian and other animal systems where uncomplexed poly I:C is not an efficient inducer of interferon. For example, in grivet monkeys, poly I:C/poly-L-lysine injected intravenously at one milligram per kilogram weight equivalent of poly I:C results in the induction of high titered circulating interferon. At a level of 0.25 mg equivalent of poly I:C substantial circulating interferon titers are achieved. Uncomplexed poly I:C injected at these dosage levels stimulates low or no detectable serum interferon.

Although the complex of this invention has not yet been administered to humans, by analogy to other similar compositions, the preferred dosage range for humans of the poly I:C/poly-L-lysine complex may be as high as 0.3—0.4 mg/kg body weight, administered as for instance on a daily basis by IV injection. Initial doses can be as low as 10 $\mu$g/kg body weight daily.

Preferred Embodiments

Example 1

Preparation of solutions of poly I:C and poly-L-lysine hydrobromide (Lot 827)

From the calculated extinction coefficient a solution containing 2.16 mg/ml of polyriboinosinic acid (poly I) as defined above in phosphate buffered saline, was obtained by heating in an 80°C water bath. The heated solution was sterilized by filtration through a 0.45 $\mu$ membrane.

From the calculated extinction coefficient a solution containing 2 mg/ml of polyribocytidylic acid (poly C), as defined above in phosphate buffered saline, was obtained by stirring at ambient temperature. The poly C solution was sterilized by filtration through a 0.45 $\mu$ filter membrane.

Equal volumes of poly I and poly C were mixed with stirring in an 80°C water bath until a clear solution was obtained. The mixture was then allowed to cool slowly at ambient temperature in order to anneal the poly I and poly C with the formation of poly I:C.

A solution of poly-L-lysine hydrobromide was prepared to contain on a dry weight basis 0.62 mg/ml in phosphate buffered saline by stirring into solution at ambient temperature. The clear solution was sterilized by filtration through a 0.2 $\mu$ membrane. The preparation of poly-L-lysine which was used as demonstrated by appropriate analytical procedures to contain equimolar content of L-lysine and bromine (the poly-L-lysine hydrobromide should be construed to have a composition as follows: [lysine·HBr]$_n$—where n is the degree of polymerization), and a molecular weight as defined.

Example 2

Preparation of poly I:C/poly-L-lysine (lot 827)

Equal volumes of poly I:C (2.08 mg/ml) prepared as set forth in Example 1 and poly-L-lysine hydrobromide (0.62 mg/ml) prepared as set forth in Example 1 were mixed with stirring. Stirring was continued for 48—72 hours at 2—8°C until only a trace of undissolved material remained. The viscous solution was clarified by filtration through a sterile clarifying membrane. The filtered solution was dispensed into ampoules and kept at 2—8°C until used. The filtered solution was demonstrated by appropriate analytical procedures to contain greater than 98% of the nominal concentration of complexed poly I:C/poly-L-lysine hydrobromide. All of the poly-L-lysine was demonstrated by appropriate sendimentation experiments to be bound to the poly I:C. The ribonuclease resistance of the poly I:C in the complex was increased 5—15 fold over the parent poly I:C. The thermal transition midpoint (Tm) was increased from 64°C (for the poly I:C alone) to 82—83°C for the complex when measured in 0.15 molar NaCl. The measurements were made by appropriate standard spectrophotometric measurements of hyperchromicity.

The following summarizes the physical characteristics of this preparation of poly I:C/poly-L-lysine, Lot 827:

| Ultraviolet spectrum | | Satisfactory |
|---|---|---|
| Absorption maximum | | 248, 265 nm |
| Absorption minimum | | 230 nm |
| Extinction coefficient (E$_{1\%}$) at 265 nm | | 140 |
| Thermal denaturation point (Tm) | | 83°C |
| Hyperchromicity on thermal denaturation | at 64°C | <10% |
| | at 83°C | 109% |
| Sedimentation coefficient (Sw, 20) | | 11.5 |
| Relative viscosity | | 1.65 |
| Nominal measure poly I:C concentration | | 1.0 mg/ml |
| Nominal poly-L-lysine concentration | | 0.310 mg/ml |

Example 3

Interferon induction in grivet monkeys (cercopithecus aethiops)

Poly I:C alone or combined with poly-L-lysine as set forth in Examples 1 and 2 were prepared and injected intravenously at 1.0 mg (as poly I:C)/kg body weight into grivet monkeys. Blood samples were obtained from animals prior to injection (prebled at time 0 hours) and at intervals thereafter. Interferon titers were determined by assay of serial dilutions of serum samples for reduction of infection of cell cultures by vesicular stomatitis virus. Peak interferon titers were obtained at approximately eight hours after injection. Characterization of the poly I:C complexes included measurement of the resistance to hydrolysis by pancreatic ribonuclease and the thermal transition midpoint (Tm) (e.g., the temperature at which half of the poly I:C has separated into the individual polynucleotides, poly I and poly C) as evidenced by hyperchromicity. Data are presented in Table 1. Peak interferon titers at least 10—100 fold greater than those obtained from monkeys induced with poly I:C alone, were obtained from monkeys induced with poly IC; complexed with poly-L-lysine.

4

# 0 025 766

Example4

Comparison of interferon induction in grivet monkeys using poly I:C complexed with poly-L-lysines of various molecular weights

Complexes of poly I:C/poly-L-lysine · HBr were prepared using samples of poly-L-lysine · HBr of defined molecular weight ranges. These complexes were compared for their capacity to induce interferon in grivet monkeys as described in Example 3. Ribonuclease sensitivity and thermal transition midpoints were determined for each of the complexes where feasible. All monkeys were injected intravenously with 1 mg (as poly I:C)/kg body weight. Samples of blood were taken immediately prior to and 8 hours post injection. Serum prepared from these samples were assayed for interferon. Resistance to ribonuclease was significantly increased in all samples measured including the molecular weight range from 1050 through approximately 9000—10,000. The thermal transition midpoint was significantly raised at all molecular weights of poly-L-lysine. Interferon induction was enhanced only marginally (geometric means titer range 50—200) with poly-L-lysines of molecular weights up through approximatley 10,000. At molecular weights above 10,000 there was a sharp rise in enhancement of interferon induction with a range of geometric mean interferon titers of 529 to 1988. These data are presented in Table 2.

## TABLE 1
### Induction of interferon in grivet monkeys

| Inducer | Ratio (wt/wt) poly I:C/ poly-L-lysine hydrobromide | Relative RNase sensitivity | Thermal transition midpoint $(Tm)^a$ | Geometric mean titer international units/ml serum interferon titer | |
|---|---|---|---|---|---|
| | | | | 0 hrs. | 8 hrs. |
| Poly I:C | — | 100 | 64°C | 10 | 48 |
| Poly I:C. poly-L-lysine hydrobromide | 1:0.31 | 16 | 82 | 9 | 1550 |

$^a$ solvent 0.15 m NaCl—0.006 M phosphate buffer, pH 7.2

## TABLE 2

| Poly I:C/poly-L-lysine · HBr interferon inducer | | | Grivet monkey geometric mean interferon titer (International units/ml) time (hours) | | |
|---|---|---|---|---|---|
| Poly-L-lysine · HBr mol. wt. | Relative RNase sensitivity | Thermal transition midpoint (°C) | $T_0$ | $T_8$ | $T_8$—$T_0$ |
| — | 100 | 64.0 | 10 | 48 | 38 |
| 1050 | 20.8 | 66.0 | 7 | 80 | 73 |
| 3760 | 2.8 | 78.5 | 29 | 230 | 201 |
| 4590 | 3.9 | 80.5 | 10 | 160 | 150 |
| 5850 | 3.2 | 80.5 | 5 | 56 | 51 |
| 9—10000 | 21.6 | 81.0 | 10 | 112 | 102 |
| 5—15000 | 18.0 | 74.0 | 20 | 1290 | 1270 |
| 20—22000 | — | — | 20 | 910 | 890 |
| 15—30000 | 11.4 | 81.0 | 12 | 2000 | 1988 |
| 32500 | 16.0 | 82.0 | 9 | 1550 | 1541 |
| >20000 | — | — | 7 | 536 | 529 |
| >70000 | 14.3 | 85.0 | 5 | 1300 | 1295 |

**Claims for the Contracting States BE CH DE FR GB IT LI LU NL SE**

1. An interferon-inducing composition comprising a solution in a pharmaceutically acceptable aqueous carrier, of a complex of polyriboinosinic-polyribocytidylic acid/poly-L-lysine hydrobromide or hydrochloride, characterized in that the concentration in solution of the polyriboinosinic-polyribocytidylic acid is 1 to 2 mg/ml, the ratio by dry weight of the polyriboinosinic-polyribocytidylic acid to the poly-L-lysine hydrobromide or hydrochloride is 1 to 0,31, or a molar ratio of 2:1; the polyriboinosinic acid has a sedimentation coefficient of 11—20, and $E_{1\%}$ at 248 nm of 240—280; the polyribocytidylic acid has a sedimentation coefficient of 5—7 and an $E_{1\%}$ at 248 nm of 175—185; and the poly-L-lysine has a molecular weight of from 10,000 to 70,000 daltons.

2. The composition of claim 1 in which the polyriboinosinic-polyribocytidylic acid concentration is 1 mg/ml.

3. The composition of claim 1 in which the poly-L-lysine is in the hydrobromide form.

4. The composition of claim 3 in which the molecular weight of the poly-L-lysine hydrobromide is from 10,000 to 35,000 daltons.

5. The composition of claim 1 wherein the polyriboinosinic acid has a sedimentation coefficient of 19 and an $E_{1\%}$ at 248 nm of 240.

6. The composition of claim 1 wherein polyribocytidylic acid has a sedimentation coefficient of 5 and an $E_{1\%}$ at 248 nm of 177.

**Claims for Contracting State AT**

1. A process for preparing an interferon-inducing composition comprising a solution in a pharmaceutically acceptable aqueous carrier, of a complex of polyriboinosinic-polyribocytidylic acid/poly-L-lysine hydrobromide or hydrochloride, said process comprising mixing: a solution of polyriboinosinic-polyribocytidylic acid with a solution of poly-L-lysine hydrobromide or hydrochloride, characterized in that the concentration in solution of the polyriboinosinic-polyribocytidylic acid is 1 to 2 mg/ml, the ratio by dry weight of the polyriboinosinic-polyribocytidylic acid to the poly-L-lysine hydrobromide or hydrochloride is 1 to 0,31, or a molar ratio of 2:1; the polyriboinosinic acid has a sedimentation coefficient of 11—20, and $E_{1\%}$ at 248 nm of 240—280; the polyribocytidylic acid has a sedimentation coefficient of 5—7 and an $E_{1\%}$ at 248 nm of 175—185; and the poly-L-lysine has a molecular weight of from 10,000 to 70,000 daltons.

2. The process of claim 1 in which the polyriboinosinic-polyribocytidylic acid concentration is 1 mg/ml.

3. The process of claim 1 in which the poly-L-lysine is in the hydrobromide form.

4. The process of claim 3 in which the molecular weight of the poly-L-lysine hydrobromide is from 10,000 to 35,000 daltons.

5. The process of claim 1 wherein the polyriboinosinic acid has a sedimentation coefficient of 19 and an $E_1$ at 248 nm of 240.

6. The process of claim 1 wherein polyribocytidylic acid has a sedimentation coefficient of 5 and an $E_{1\%}$ at 248 nm of 177.

**Patentansprüche für die Vertragsstaaten BE CH DE FR GB IT LI LU NL SE**

1. Interferon-induzierende Zusammensetzung enthaltend eine Lösung in einem pharmazeutisch brauchbaren wäßrigen Träger von einem Komplex von Polyriboinosin-Polyribocytidyl-säure/Poly-L-lysin-hydrobromid oder -hydrochlorid, dadurch gekennzeichnet, daß die Konzentration in Lösung von der Polyriboinosin-Polyribocytidyl-säure 1 bis 2 mg/ml beträgt, das Verhältnis von Trockengewicht der Polyriboinosin-Polyribocytidyl-säure zu dem Poly-L-lysin-hydrobromid oder -hydrochlorid 1 bis 0,31 beträgt oder ein Molverhältnis von 2:1 ist; die Polyriboinosin-säure einen Sedimentationskoeffizienten von 11—20 und ein $E_{1\%}$ bei 248 nm von 240—280 aufweist; die Polyribocytidyl-säure einen Sedimentationskoeffizienten von 5—7 und ein $E_{1\%}$ bei 248 nm von 175—185 aufweist; und das Poly-L-lysin ein Molekulargewicht von 10 000 bis 70 000 Dalton aufweist.

2. Zusammensetzung nach Anspruch 1, in der die Polyriboinosin-Polyribocytidyl-säure-Konzentration 1 mg/ml beträgt.

3. Zusammensetzung nach Anspruch 1, in der das Poly-L-lysin in der Hydrobromidform vorliegt.

4. Zusammensetzung nach Anspruch 3, in der das Molekulargewicht des Poly-L-lysin hydrobromid 10 000 bis 35 000 Dalton beträgt.

5. Zusammensetzung nach Anspruch 1, in der die Polyriboinosin-säure einen Sedimentationskoeffizienten von 19 und ein $E_{1\%}$ bei 248 nm von 240 aufweist.

6. Zusammensetzung nach Anspruch 1, in der die Polyribocytidyl-säure einen Sedimentationskoeffizienten von 5 und ein $E_{1\%}$ bei 248 nm von 177 aufweist.

**Patentansprüche für den Vertragsstaat AT**

1. Ein Verfahren zur Herstellung einer interferoninduzierenden Zusammensetzung, umfassend

## 0 025 766

eine Lösung eines Komplexes von Polyriboinosin-Polyribocytidylsäure/Poly-L-lysinhydrobromid oder -hydrochlorid in einem pharmazeutisch annehmbaren wässerigen Träger, welches Verfahren des Mischen einer Lösung von Polyriboinosin-Polyribocytidylsäure mit einer Lösung von Poly-L-lysinhydrobromid oder -hydrochlorid umfaßt, dadurch gekennzeichnet, daß die Konzentration der Polyriboinosin-Polyribocytidylsäure in Lösung 1 bis 2 mg/ml beträgt, das Trockengewichtsverhältnis der Polyriboinosin-Polyribocytidylsäure zu dem Poly-L-lysinhydrobromid oder -hydrochlorid 1 bis 0,31, oder ein Molverhältnis von 2:1 ist; die Polyriboinosinsäure einen Sedimentationskoeffizienten von 11— 20 und eine $E_{1\%}$ bei 248 nm von 240—280 hat; die Polyribocytidylsäure einen Sedimentationskoeffizienten von 5—7 und eine $E_{1\%}$ bei 248 nm von 175—185 hat; und das Poly-L-lysin ein Molekulargewicht von 10.000 bis 70.000 Dalton hat.

2. Das Verfahren des Anspruchs 1, in welchem die Polyriboinosin-Polyribocytidylsäure-konzentration 1 mg/ml beträgt.

3. Das Verfahren des Anspruchs 1, in welchem das Poly-L-lysin in der Hydrobromidform vorliegt.

4. Das Verfahren des Anspruchs 3, in welchem das Molekulargewicht des Poly-L-lysinhydrobromids 10.000 bis 35.000 Dalton beträgt.

5. Das Verfahren des Anspruchs 1, worin die Polyriboinosinsäure einen Sedimentationskoeffizienten von 19 und eine $E_{1\%}$ bei 248 nm von 240 hat.

6. Das Verfahren des Anspruchs 1, worin die Polyribocytidylsäure einen Sedimentationskoeffizienten von 5 und eine $E_{1\%}$ bei 248 nm von 177 hat.

### Revendications pour les Etats contractants BE CH DE FR GB IT LI LU NL SE

1. Procédé de préparation d'une composition inductrice d'interféron comprenant une solution dans un support aqueux acceptable en pharmacie d'un complexe d'acide polyriboinosinique-polyribocytidylique/bromhydrate ou chlorhydrate de poly-L-lysine, ce procédé comprenant le mélange d'une solution d'acide polyriboinosinique-polyribocytidylique avec une solution de bromhydrate ou chlorhydrate de poly-L-lysine, caractérisé en ce que la concentration en solution de l'acide polyriboinosinique-polyribocytidylique est de 1 à 2 mg/ml, le rapport en poids sec de l'acide polyriboinosinique-polyribocytidylique au bromhydrate ou chlorhydrate de poly-L-lysine est de à 0,31 soit un rapport molaire de 2/1; l'acide polyribonosinique a un coefficient de sédimentation de 11—20 et un $E_{1\%}$ à 248 nm de 240—280; l'acide polyribocytidylique a un coefficient de sédimentation de 5— 7 et un $E_{1\%}$ à 248 nm de 175—185; et la poly-L-lysine a un poids moléculaire de 10 000 à 70 000 daltons.

2. Le procédé de la revendication 1 dans lequel la concentration de l'acide polyriboinosinique-polyribocytidylique est de 1 mg/ml.

3. Le procédé de la revendication 1 dans lequel la poly-L-lysine est sous forme du bromhydrate.

4. Le procédé de la revendication 3 dans lequel le poids moléculaire du bromhydrate de poly-L-lysine est de 10 000 à 35 000 daltons.

5. Le procédé de la revendication 1 dans lequel l'acide polyriboinosinique a un coefficient de sédimentation de 19 et un $E_{1\%}$ à 248 nm de 240.

6. Le procédé de la revendication 1 dans lequel l'acide polyribocytidylique a un coefficient de sédimentation de 5 et un $E_{1\%}$ à 248 nm de 177.

### Revendications pour l'Etat contractant AT

1. Une composition inductrice d'interféron comprenant une solution dans un support aqueux acceptable en pharmacie d'un complexe d'acide polyriboinosinique-polyribocytidylique/bromhydrate ou chlorohydrate de poly-L-lysine, caractérisée en ce que la concentration en solution de l'acide polyriboinosinique-polyribocytidylique est de 1 à 2 mg/ml, le rapport en poids sec de l'acide polyriboinosinique-polyribocytidylique au bromhydrate ou chlorhydrate de poly-L-lysine est de 1 à 0,31 soit un raport molaire de 2/1; l'acide polyriboinosinique a un coefficient de sédimentation de 11—20 et un $E_{1\%}$ à 248 nm de 240—280; l'acide polyribocytidylique a un coefficient de sédimentation de 5—7 et un $E_{1\%}$ à 248 nm de 175—185; et la poly-L-lysine a un poids moléculaire de 10 000 à 70 000 daltons.

2. La composition de la revendication 1 dans laquelle la concentration de l'acide polyriboinosinique-polyribocytidylique est de 1 mg/ml.

3. La composition de la revendication 1 dans laquelle la poly-L-lysine est sous forme du bromhydrate.

4. La composition de la revendication 3 dans laquelle le poids moléculaire du bromhydrate de poly-L-lysine est de 10 000 à 35 000 daltons.

5. La composition de la revendication 1 dans laquelle l'acide polyriboinosinique a un coefficient de sédimentation de 19 et un $E_{1\%}$ à 248 nm de 240.

6. La composition de la revendication 1 dans laquelle l'acide polyribocytidylique a un coefficient de sédimentation de 5 et un $E_{1\%}$ à 248 nm de 177.